# EUROPÄISCHE PATENTANMELDUNG

(11) **EP 4 302 712 A2**
(43) Veröffentlichungstag der Anmeldung: **10.01.2024**
(21) Anmeldenummer: 23212200.2
(22) Anmeldetag: 30.11.2020
(51) Int. Cl.: A61B 17/88

(54) **VORRICHTUNG UND VERFAHREN ZUM PRÄPARIEREN EINES KNOCHENTRANSPLANTATS**

(30) Priorität: 29.11.2019 DE 102019132412
(62) Teilanmeldung aus: 20817242.9
(71) Anmelder: Mathys AG Bettlach, 2544 Bettlach (CH)
(72) Erfinder: Hesky, Claudia, 07743 Jena (DE); Dallmann, Frank, 04626 Schmölln (DE); Settke, Alexander, 07749 Jena (DE)
(74) Vertreter: Körfer, Thomas

(57) **Zusammenfassung**

Die erfindungsgemäße Vorrichtung zum Präparieren eines Knochentransplantats (100) außerhalb des menschlichen oder tierischen Körpers besteht aus einem Grundkörper (101) und zumindest einem Halteelement (102). Das zumindest eine Halteelement (102) ist in den Grundkörper (101) der Vorrichtung zum Präparieren eines Knochentransplantats (100) eingesetzt. Im erfindungsgemäßen Verfahren zum Konfigurieren der erfindungsgemäßen Vorrichtung wird das zumindest eine Halteelement (102) in den Grundkörper der Vorrichtung zum Präparieren eines Knochentransplantats (100) eingesetzt. Im erfindungsgemäßen Verfahren zum Präparieren eines Knochentransplantats außerhalb des menschlichen oder tierischen Körpers mit der erfindungsgemäßen Vorrichtung (100) wird ein zu präparierendes Knochenstück in das im Grundkörper (100) eingesetzte Halteelement (102) eingelegt, das zu präparierende Knochenstück mit dem eingesetzten Halteelement (102) fixiert, und das zu präparierende Knochenstück bearbeitet.

## Beschreibung

Die Erfindung betrifft eine Vorrichtung zum Präparieren eines Knochentransplantats zum Einsetzen an einen menschlichen oder tierischen Knochen und ein zugehöriges Verfahren zum Konfigurieren der Vorrichtung.

Krankheiten, Unfälle oder auch Verschleißerscheinungen durch Überbelastung können Gelenke des menschlichen oder des tierischen Körpers schädigen. Lassen sich die Gelenke durch konservative Methoden nicht ausheilen, kann es notwendig sein, das Gelenk zu ersetzen. Beim menschlichen Körper ist es häufig erforderlich, geschädigte Schultergelenke, Hüftgelenke oder Kniegelenke durch Gelenk-Implantate zu ersetzen. Entsprechend des befallenen Gelenks sind verschiedene Methoden zur Implantation von künstlichen Gelenken verfügbar. Im Bereich der Schultergelenke kommt teilweise die sogenannte inverse Schulterprothese zum Einsatz. Inverse Schulterprothesen sind dadurch gekennzeichnet, dass die künstliche Gelenkpfanne den geschädigten Humeruskopf und die künstliche Gelenkkugel das geschädigte Glenoid ersetzt.

Eine Besonderheit moderner inverser Schulterprothesen ist die Verwendung von Knochentransplantaten im Bereich des Glenoids. Die Verwendung eines Knochentransplantats ermöglicht die sichere Fixation eines Implantats trotz Knochenverlust.

Im Allgemeinen wird das verwendete Knochentransplantat aus dem Humeruskopf der operierten Schulter gewonnen. Hierzu wird ein Führungsdraht mit Hilfe eines Positionierinstruments im Humeruskopf platziert. In einem nächsten Schritt wird die Oberfläche des Knochentransplantats plangefräst. Hierbei werden Knorpelreste und die harten Knochenanteile des Humeruskopfes entfernt. Mit einem Kronenbohrer wird der zu extrahierende Knochen an seinen Seitenrändern vom Humeruskopf getrennt. Nun wird eine Bohrung in das zu extrahierende Knochenstück im Bereich des Führungsdrahts gebohrt, wodurch gleichzeitig der Führungsdraht entfernt wird. Das Trennen des zu extrahierenden Knochenstücks an seinen Seitenrändern und das Bohren kann unter Verwendung eines Kronenbohrers mit integriertem Zentralbohrer in einem Arbeitsschritt erfolgen. Im letzten Schritt der Extraktion kommt ein Schnittführungsinstrument zum Einsatz. Dieses Schnittführungsinstrument ist so gestaltet, dass es genau passend auf die gefrästen Oberflächen aufgesetzt wird. In einem an dem Schnittführungsinstrument angebrachten Schlitz wird eine Knochensäge so geführt, dass das zu extrahierende Knochentransplantat mit einem vorgegebenen Winkel reseziert wird. Weitere Varianten von Schnittführungswerkzeugen enthalten ein Plateau zur Führung der Knochensäge. Hierbei wird die Knochensäge durch ein einseitiges Aufliegen auf dem Plateau geführt.

Die US 2019/0209186 A1 beschreibt ein solches Schnittführungsinstrument zum Extrahieren eines Knochentransplantats. Das Schnittführungsinstrument enthält eine Grundplatte, die auf das vorbereitete zu extrahierende Knochenteil aufgesetzt wird. Außerdem enthält das Schnittführungsinstrument eine Sägeführung, die dazu ausgelegt ist, eine Schnittklinge einer Knochensäge aufzunehmen und zu führen. Diese Sägeführung ist über einen Tiefenschenkel mit der Grundplatte beabstandet verbunden.

Nachteilig an diesem Schnittführungsinstrument zum Extrahieren eines Knochentransplantats ist, dass das Schnittführungsinstrument im Körper des Patienten Anwendung findet. Durch die Art der Führung des Sägeblatts, insbesondere der einseitigen Führung, ist eine Maßhaltigkeit des extrahierten Knochentransplantats gering.

Der Erfindung liegt daher die Aufgabe zugrunde, eine Vorrichtung zum Extrahieren eines Knochentransplantats eines menschlichen oder tierischen Knochens und ein zugehöriges Verfahren zum Konfigurieren der Vorrichtung zu schaffen, ohne die aufgeführten Nachteile in Kauf nehmen zu müssen.

Die Aufgabe wird in vorteilhafter Weise durch eine erfindungsgemäße Vorrichtung zum Präparieren eines Knochentransplantats außerhalb des menschlichen oder tierischen Körpers gemäß Anspruch 1 und durch ein Verfahren zum Konfigurieren der Vorrichtung außerhalb des menschlichen oder tierischen Körpers gemäß Anspruch 11 gelöst. Vorteilhafte Weiterbildungen sind Gegenstand der hierauf rückbezogenen Unteransprüche.

Die erfindungsgemäße Vorrichtung zum Präparieren eines Knochentransplantats außerhalb des menschlichen oder tierischen Körpers besteht aus einem Grundkörper und zumindest einem Halteelement. Das zumindest eine Halteelement ist in den Grundkörper der Vorrichtung zum Präparieren eines Knochentransplantats eingesetzt.

Ein Knochentransplantat, das vorher dem Körper entnommen wurde, kann in besonders vorteilhafter Weise durch das im Grundkörper befindliche Haltelement zur Präparation festgehalten werden. Durch das Halteelement wird ein sicheres Halten des Knochentransplantats in einer exakt festgelegten Position erreicht. Erst das sichere Halten des Knochentransplantats mit der Haltevorrichtung ermöglicht ein äußerst präzises Bearbeiten des Knochentransplantats.

Vorteilhafterweise weist die Vorrichtung zum Präparieren eines Knochentransplantats vorzugsweise ein erstes Halteelement und ein zweites Halteelement auf, wobei das erste Halteelement ein Führungseinsatz und das zweite Halteelement ein Halteeinsatz ist. Besonders vorteilhaft wird das zu präparierende Knochentransplantat durch den Führungseinsatz sicher in seiner Position geführt und durch den Halteeinsatz in dieser Position fixiert.

In vorteilhafter Weise weist der Grundkörper bevorzugt
mindestens einen Schlitz zur Führung eines spanabhebenden Werkzeugs auf. Mithilfe des mindestens einen Schlitzes wird das spanabhebende Werkzeug so geführt, dass die durch den Schlitz vorgegebene Lage und der Winkel exakt eingehalten werden.

Vorteilhafterweise weist der Führungseinsatz eine Anschlagfläche auf, die bevorzugt einen Winkel bezogen auf den Schlitz zur Führung des spanabhebenden Werkzeugs aufweist. Der Halteeinsatz weist bevorzugt einen zum Winkel des Führungseinsatzes korrespondierenden Winkel bezogen auf den Schlitz zur Führung des spanabhebenden Werkzeugs auf. Die im Winkel angeordnete Anschlagfläche des Führungseinsatzes ermöglicht die präzise Bearbeitung des extrahierten Knochenstücks unter Einhaltung des vorgegebenen Winkels. Durch den korrespondierenden Winkel des Halteeinsatzes ist es möglich, das Knochenstück so zu fixieren, dass während des gesamten Schnittvorgangs keine Winkeländerung auftreten kann.

Vorteilhafterweise weist der Führungseinsatz bevorzugt eine weitere Anschlagfläche mit einem weiteren unterschiedlichen Winkel bezogen auf den Schlitz zur Führung des spanabhebenden Werkzeugs auf. Außerdem weist der Halteeinsatz bevorzugt einen weiteren zum weiteren Winkel des Führungseinsatzes korrespondierenden Winkel bezogen auf den Schlitz zur Führung des spanabhebenden Werkzeugs auf. Somit ist es möglich, mit nur einer Vorrichtung zum Präparieren eines Konchentransplantats, Knochenstücke mit verschiedenen Schnittwinkeln anzufertigen.

In vorteilhafter Weise sind die Anschlagfläche und die weitere(n) Anschlagfläche(n) des Führungseinsatzes bevorzugt gegenüberliegend angeordnet. Eine solche Anordnung erlaubt es dem Benutzer, die Schnittparameter durch ein einfaches Wenden des Führungseinsatzes an die Vorgaben der Prothese anzupassen.

Vorteilhafterweise umfasst der Halteeinsatz bevorzugt mindestens zwei Führungen, wobei die mindestens zwei Führungen geometrisch unterschiedlich ausgeführt sind. Der Grundkörper enthält korrespondierend hierzu bevorzugt mindestens zwei Führungsaufnahmen zur Aufnahme der Führungen des Halteeinsatzes. Durch die unterschiedliche Ausgestaltung der zwei Führungen ist es ausgeschlossen, dass der Halteeinsatz fehlerhaft eingesetzt wird. Die Prozesssicherheit wird dadurch erheblich verbessert.

Es ist weiterhin von Vorteil, dass der Halteeinsatz bevorzugt mindestens eine Fingermulde enthält. Die Fingermulde ermöglicht es, eine höhere Schubkraft in den Halteeinsatz einzuleiten. Somit wird eine zuverlässige Haltekraft auf das Knochentransplantat ausgeübt. Außerdem ist die Gefahr des Abrutschens der Finger des Präparators durch die Fingermulde deutlich reduziert.

Vorteilhafterweise enthält der Führungseinsatz bevorzugt mindestens eine halbkreisförmige bzw. halbzylinderförmige Ausnehmung zur Aufnahme des Knochentransplantats, deren Achse rechtwinklig zur Anschlagfläche des Führungseinsatzes angeordnet ist. Damit ist ein sicheres Platzieren des extrahierten Knochenstücks in der erforderlichen Lage in der Vorrichtung zum Präparieren eines Knochentransplantats gewährleistet.

Es ist weiterhin von Vorteil, wenn der Führungseinsatz bevorzugt eine weitere halbkreisförmige bzw. halbzylinderförmige Ausnehmung zur Aufnahme des Knochentransplantats enthält, deren Achse rechtwinklig zur weiteren Anschlagfläche des Führungseinsatzes angeordnet ist. Durch diese Anordnung lassen sich Knochentransplantate mit einer unterschiedlichen Geometrie präparieren, indem der Führungseinsatz entsprechend in den Grundkörper eingesetzt wird.

Im erfindungsgemäßen Verfahren zum Konfigurieren der Vorrichtung zum Präparieren eines Knochentransplantats außerhalb des menschlichen oder tierischen Körpers wird das zumindest eine Halteelement in den Grundkörper der Vorrichtung zum Präparieren eines Knochentransplantats eingesetzt.

Es ist weiterhin von Vorteil, wenn im Verfahren zum Konfigurieren einer Vorrichtung zum Präparieren eines Knochentransplantats bevorzugt ein Führungseinsatz des ersten Halteelements in den Grundkörper eingesetzt wird und dass ein Halteeinsatz des zweiten Halteelements in den Grundkörper eingesetzt wird. Dadurch lässt sich die Vorrichtung zum Präparieren eines Knochentransplantats in einer an das Implantat angepassten Konfiguration zusammensetzen.

Somit kann beim Präparieren eines Knochentransplantats außerhalb des menschlichen oder tierischen Körpers mit der erfindungsgemäßen Vorrichtung ein zu präparierendes Knochenstück in das im Grundkörper zumindest eine eingesetzte Halteelement eingelegt werden, das zu präparierende Knochenstück mit dem zumindest einen eingesetzten Haltelement fixiert werden und
das zu präparierende Knochenstück bearbeitet werden. Dadurch lässt sich das Knochentransplantat besonders schonend und exakt für den Patienten anfertigen.

Ausführungsbeispiele der erfindungsgemäßen Vorrichtung zum Präparieren eines Knochentransplantats außerhalb des menschlichen oder tierischen Körpers und des zugehörigen Verfahrens sind in den Zeichnungen beispielhaft dargestellt und werden anhand der nachfolgenden Beschreibung näher erläutert. Die Zeichnungen zeigen:
- FIG. 1: ein Ausführungsbeispiel einer erfindungsgemäßen Vorrichtung zum Präparieren eines Knochentransplantats mit einem Halteelement in perspektivischer Ansicht;
- FIG. 2: ein zweites Ausführungsbeispiel der erfindungsgemäßen Vorrichtung zum Präparieren eines Knochentransplantats mit einem Führungseinsatz und einem Halteeinsatz in perspektivischer Ansicht;
- FIG. 3: den Grundkörper des zweiten Ausführungsbeispiels der Vorrichtung zum Präparieren eines Knochentransplantats im Dreitafelbild;
- FIG. 4: den Führungseinsatz des zweiten Ausführungsbeispiels der Vorrichtung zum Präparieren eines Knochentransplantats im Dreitafelbild;
- FIG. 5: den Führungseinsatz des zweiten Ausführungsbeispiels der Vorrichtung zum Präparieren eines Knochentransplantats in einer Schnittdarstellung;
- FIG. 6: den Halteeinsatz des zweiten Ausführungsbeispiels der Vorrichtung zum Präparieren eines Knochentransplantats im Dreitafelbild;
- FIG. 7: den Halteeinsatz des zweiten Ausführungsbeispiels der Vorrichtung zum Präparieren eines Knochentransplantats in einer Schnittdarstellung;
- FIG. 8: das zweite Ausführungsbeispiel der erfindungsgemäßen Vorrichtung zum Präparieren eines Knochentransplantats mit einem Führungseinsatz und einem Halteeinsatz in einer Explosionsdarstellung;
- FIG. 9: das zweite Ausführungsbeispiel der erfindungsgemäßen Vorrichtung zum Präparieren eines Knochentransplantats mit einem Führungseinsatz und einem Halteeinsatz und eingelegtem Knochentransplantat in Draufsicht;
- FIG. 10: das zweite Ausführungsbeispiel der erfindungsgemäßen Vorrichtung zum Präparieren eines Knochentransplantats mit einem Führungseinsatz und einem Halteeinsatz und eingelegtem Knochentransplantat in Schnittdarstellung;
- FIG. 11: den Führungseinsatz des zweiten Ausführungsbeispiels der Vorrichtung zum Präparieren eines Knochentransplantats mit einem Knochentransplantat in einer perspektivischen Ansicht;
- FIG. 12: ein drittes Ausführungsbeispiel der erfindungsgemäßen Vorrichtung zum Präparieren eines Knochentransplantats mit einem Führungseinsatz und einem Halteeinsatz im Dreitafelbild;
- FIG. 13: ein Grundkörper eines vierten Ausführungsbeispiels der Vorrichtung zum Präparieren eines Knochentransplantats im Dreitafelbild; und
- FIG. 14: die Anwendung der erfindungsgemäßen Vorrichtung zum Präparieren eines Knochentransplantats.

Einander entsprechende Teile sind in allen Figuren mit den gleichen Bezugszeichen versehen.

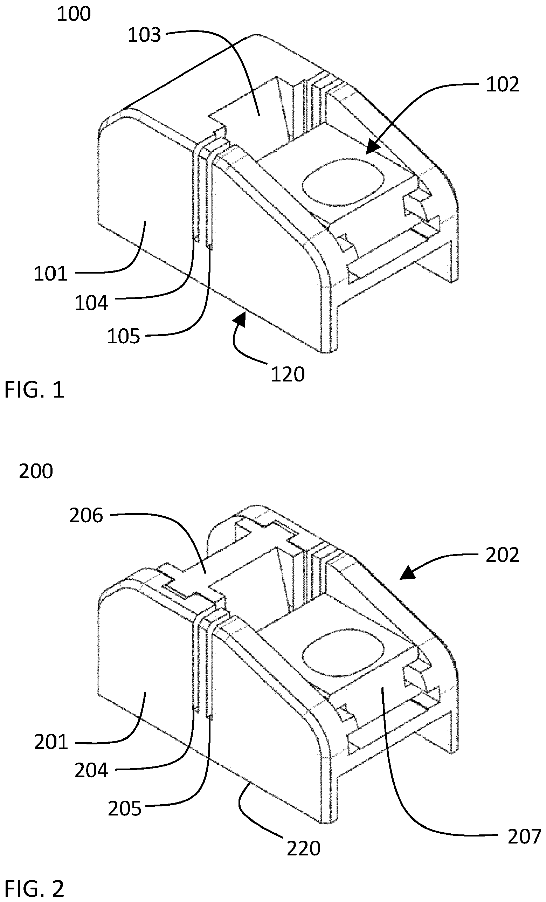

FIG. 1 zeigt ein Ausführungsbeispiel einer erfindungsgemäßen Vorrichtung zum Präparieren eines Knochentransplantats 100. Die Vorrichtung zum Präparieren eines Knochentransplantats 100 besteht aus einem Grundkörper 101 und einem Halteelement 102. Das Halteelement 102 ist in den Grundkörper 101 eingesetzt. In diesem Ausführungsbeispiel kann das Halteelement 102 stirnseitig in den Grundkörper 101 eingeschoben werden. Zur Aufnahme des zu präparierenden Knochenstücks ist in den Grundkörper 101 eine Anschlagsfläche 103 vorgesehen. Im Grundkörper 101 sind außerdem Schlitze 104, 105 eingeschnitten. Diese Schlitze 104, 105 verlaufen senkrecht zur Auflagefläche 120 des Grundkörpers 101. In FIG. 1 sind beispielhaft zwei Schlitze 104, 105 dargestellt. Der Grundkörper 101 kann jedoch mit einer beliebigen Anzahl von Schlitzen ausgestattet sein.

Zum Präparieren eines Knochentransplantats wird in den Grundkörper 101, entlang der Anschlagfläche 103, eine extrahierte Knochenscheibe eingeschoben. Diese extrahierte Knochenscheibe wird mit Hilfe des Halteelements 102 in ihrer Position fixiert. Je nach geforderter Scheibendicke des Knochentransplantats wird in einem der beiden Schlitze 104, 105 ein Schneidwerkzeug geführt.

Nach der Fertigstellung des Knochentransplantats kann das Halteelement 102 aus dem Grundkörper 101 entfernt werden. Die Einzelteile der Vorrichtung zum Präparieren eines Knochentransplantats 100 lassen sich so besonders leicht reinigen. Außerdem ist eine Kontrolle des Reinigungsergebnisses vereinfacht, da die Anschlagfläche 103 gut einsehbar wird.

FIG. 2 zeigt ein zweites Ausführungsbeispiel einer erfindungsgemäßen Vorrichtung zum Präparieren 200 eines Knochentransplantats. Die Vorrichtung zum Präparieren eines Knochentransplantats 200 des zweiten Ausführungsbeispiels besteht aus einem Grundkörper 201 und zwei Halteelementen 202. Das erste Halteelement 202 ist ein Führungseinsatz 206. Das zweite Halteelement 202 ist ein Halteeinsatz 207. Im Folgenden werden die Komponenten der Vorrichtung zum Präparieren eines Knochentransplantats 200 des zweiten Ausführungsbeispiels näher beschrieben.

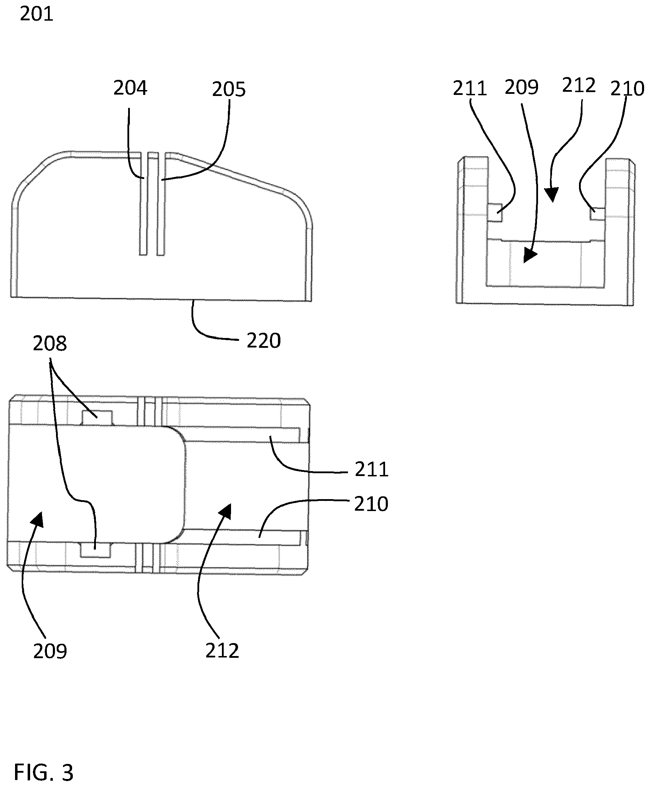

FIG. 3 zeigt einen Grundkörper des zweiten Ausführungsbeispiels einer Vorrichtung zum Präparieren eines Knochentransplantats 200 im Dreitafelbild. Zur Verbesserung der Übersichtlichkeit werden die Bezugszeichen nicht in jeder Ansicht des Dreitafelbilds wiederholt, außer es ist zum Verständnis der Zeichnungen notwendig.

Die in FIG. 3 abgebildete Vorderansicht zeigt den Grundkörper 201 mit zwei Schlitzen 204 und 205. Diese Schlitze 204, 205 verlaufen senkrecht zur Auflagefläche 220 des Grundkörpers 201. In FIG. 3 sind beispielhaft zwei Schlitze 204, 205 dargestellt. Der Grundkörper 201 können jedoch eine beliebige Anzahl von Schlitzen vorgesehen sein. Der erste Schlitz 204 kommt zum Einsatz, wenn ein dünnes Knochentransplantat erstellt werden soll. Für dicke Knochentransplantate ist der zweite Schlitz 205 vorgesehen.

Der Grundkörper 201 enthält zwei Ausnehmungen 208. Diese Ausnehmungen 208 sind in der Draufsicht des Grundkörpers 201 dargestellt. Diese Ausnehmungen 208 können nutförmig (wie in FIG. 3 abgebildet) oder halbrund ausgeführt sein. Es sind auch beliebige andere Formen der Ausnehmungen 208 denkbar. Diese Ausnehmungen 208 dienen als Führungen für den Führungseinsatz 206 (hier nicht dargestellt).

Des Weiteren enthält der Grundkörper 201 in dem Bereich, in den der Führungseinsatz 206 eingeführt werden kann, eine Vertiefung 209. Die Vertiefung 209 stellt den Platz für das Führungselement 206 (hier nicht dargestellt) in dem Grundkörper 201 zur Verfügung.

FIG. 3 zeigt zwei im Grundkörper 201 angebrachte Führungsaufnahmen 210, 211. Diese Führungsaufnahmen 210, 211 sind in Längsrichtung im Bereich einer Ausnehmung 212 zur Aufnahme des Halteeinsatzes 207 (hier nicht dargestellt) im Grundkörper 201 angebracht. Wie zu erkennen ist, unterscheiden sich die beiden Führungsaufnahmen 210, 211 in ihrer Formgebung. In dem hier dargestellten Fall ist die linke Führungsaufnahme 210 deutlich höher als die rechte Führungsaufnahme 211. Durch diese unterschiedlichen Ausführungen der beiden Führungsaufnahmen 210, 211 kann sichergestellt werden, dass der Halteeinsatz 207 (hier nicht dargestellt) ausschließlich lagerichtig in den Grundkörper 201 eingesetzt werden kann.

Der Grundkörper 101, 201 der Vorrichtung zum Präparieren eines Knochentransplantats 100, 200 besteht aus Metall oder Kunststoff. Vorzugsweise ist der Grundkörper 101, 201 aus einem Edelstahl gefertigt. Ist der Grundkörper 101, 201 aus Kunststoff gefertigt, kommt vorzugsweise ein PA (Polyamid), ein PEEK (Polyetheretherketon), ein PPSU (Polyphenylensulfon) oder ein POM-C (Polyoxymethylen Copolymer) zum Einsatz. Die Materialien sind nur beispielhaft genannt und schränken die mögliche Materialauswahl nicht ein.

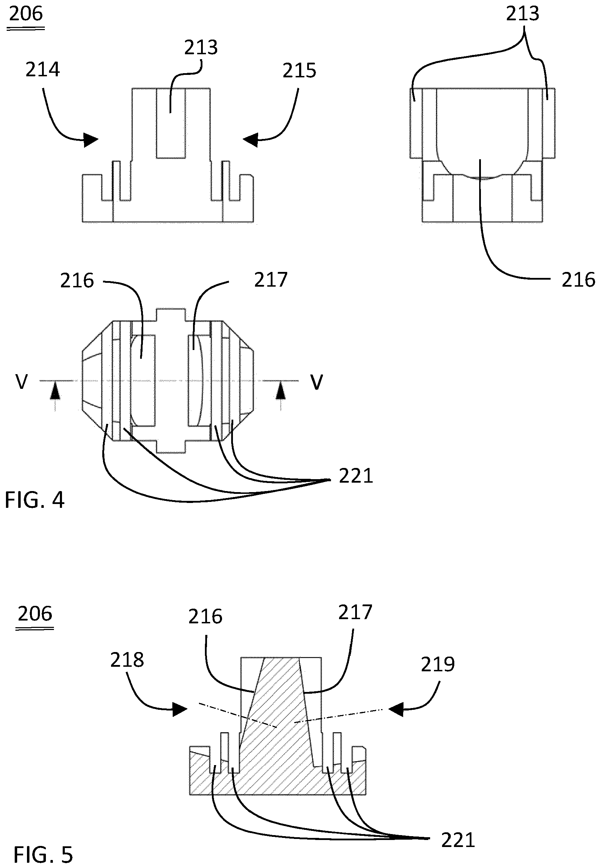

FIG. 4 zeigt einen Führungseinsatz 206 des zweiten Ausführungsbeispiels einer Vorrichtung zum Präparieren eines Knochentransplantats 200 im Dreitafelbild. Zur weiteren Veranschaulichung des Führungseinsatzes 206 zeigt FIG. 5 eine dazugehörige Schnittdarstellung, wobei die Schnittebene in FIG. 4 mit dem Schnitt V-V markiert ist.

Der Führungseinsatz 206 enthält zwei Führungsaufnahmen 213. Diese Führungsaufnahmen 213 sind so gestaltet, dass sie mit den Ausnehmungen 208 des Grundkörpers 201 eine passgenaue schiebbare Verbindung herstellen.

Zur Führung des zu präparierenden Knochenstücks enthält der Führungseinsatz 206 an zwei gegenüberliegenden Seiten je eine halbkreisförmige Ausnehmung 214, 215. Der Durchmesser der halbkreisförmigen Ausnehmung 214, 215 entspricht dem Durchmesser des zu präparierenden Knochenstücks. Falls das Knochentransplantat aus dem gesamten Humeruskopf präpariert wird, ist der Durchmesser der halbkreisförmigen Ausnehmung 214, 215 dem Durchmesser des Humeruskopfs angepasst. Die halbkreisförmigen Ausnehmungen 214, 215 enden jeweils an einer Anschlagfläche 216, 217. Die Achse 218, 219 der halbkreisförmigen Ausnehmung 214, 215 ist rechtwinklig zur zugehörigen Anschlagfläche 216, 217 ausgerichtet. Die Anschlagfläche 216, 217 des Führungseinsatzes weist einen Winkel in Bezug auf die Schlitze 204, 205 (hier nicht dargestellt) zum Führen des Schneidwerkzeugs im Grundkörper 201 auf.

Damit der Führungseinsatz 206 beim Präparieren des Knochentransplantats nicht beschädigt wird, sind Schlitze 221 in den Führungseinsatz 206 geschnitten. Die Schlitze 221 sind so angebracht, dass die Schlitze 221 in den beiden Einbaulagen des Führungseinsatzes 206 passgenau zu den Schlitzen 204, 205 (hier nicht dargestellt) des Grundkörpers 201 liegen.

Der Führungseinsatz 206 besteht aus Metall oder Kunststoff. Vorzugsweise ist der Führungseinsatz 206 aus einem Edelstahl gefertigt. Ist der Führungseinsatz 206 aus Kunststoff gefertigt, kommt vorzugsweise auch hier ein PA (Polyamid), ein PEEK (Polyetheretherketon), ein PPSU (Polyphenylensulfon) oder ein POM-C (Polyoxymethylen Copolymer)zum Einsatz. Die Materialien sind nur beispielhaft genannt und schränken die mögliche Materialauswahl nicht ein.

In FIG. 5 ist weiter deutlich zu erkennen, dass die Anschlagfläche 216, 217 des Führungseinsatzes 206 jeweils einen Winkel in Bezug auf die Schlitze zum Führen des Schneidwerkzeugs 204, 205 im Grundkörper 201 aufweist. Der Winkel der Anschlagsfläche 216, 217 kann, abhängig von der Anforderung an das Knochentransplantat, einen Winkel zwischen 0° und 45° aufweisen. Bei dem Führungseinsatz 206 in diesem Ausführungsbeispiel hat die Anschlagfläche auf der einen Seite 217 vorzugsweise einen Winkel von 7,5° und die Anschlagsfläche auf der gegenüberliegenden Seite 216 vorzugsweise einen Winkel von 15°.

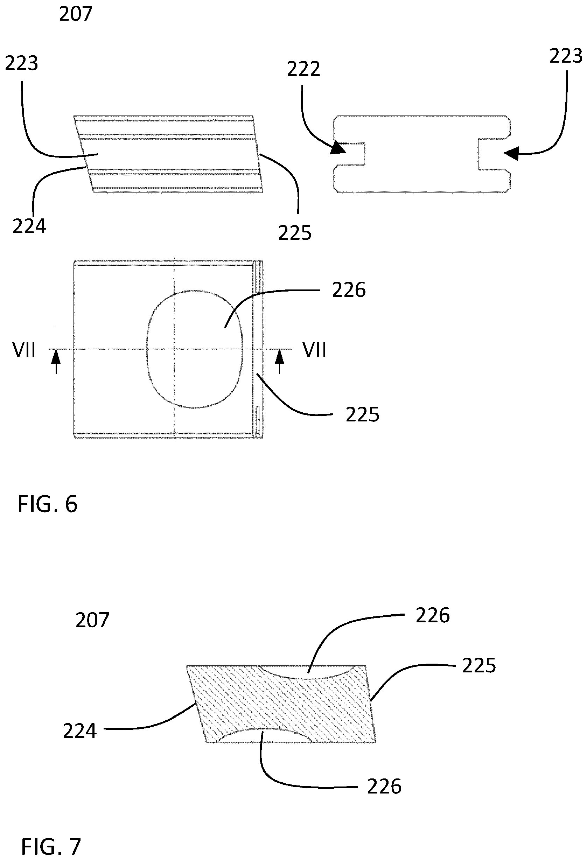

FIG. 6 und FIG. 7 zeigen einen Halteeinsatz 207 des zweiten Ausführungsbeispiels einer Vorrichtung zum Präparieren eines Knochentransplantats 200. FIG. 7 zeigt den Halteeinsatz 207 in einer Schnittdarstellung, wobei die Schnittebene ist FIG. 6 mit dem Schnitt VII-VII markiert ist. Der Halteeinsatz 207 enthält hier vorzugsweise mindestens zwei Führungen 222, 223, die geometrisch unterschiedlich geformt sind, so dass sie mit den oben beschriebenen Führungsaufnahmen 210, 211 des Grundkörpers 201 zusammen eine schiebbare Verbindung bilden. Vorzugsweise ist die schiebbare Verbindung nach dem Poka Yoke Prinzip ausgeführt. Schiebbare Verbindungen nach dem Poka Yoke Prinzip lassen sich ausschließlich lagerichtig ineinander schieben.
Die stirnseitigen Flächen 224, 225 des Halteeinsatzes 207 sind korrespondierend zum Führungseinsatz 206 mit jeweils einem Winkel in Bezug auf die Schlitze zum Führen des Schneidwerkzeugs 204, 205 im Grundkörper 201 ausgeführt. Somit kann der Winkel der stirnseitige Fläche 224, 225, abhängig vom korrespondierenden Winkel des Führungseinsatzes 206, einen Winkel zwischen 0° und 45° aufweisen. Vorzugsweise hat eine erste stirnseitige Fläche 225 einen Winkel von 7,5° und die gegenüberliegende zweite stirnseitige Fläche 224 einen Winkel von 15°.

Die Oberfläche des Halteeinsatzes 207 enthält bevorzugt mindestens eine Fingermulde 226. Die Fingermulde 226 ist so geformt, dass eine Fingerkuppe oder mehrere Fingerkuppen des Anwenders darin Platz finden. Die Fingermulde 226 kann auch so gestaltet sein, dass für größerer Haltekräfte ein Handballen oder andere geeignete Körperteile darin halt finden. Aufgabe der Fingermulden 226 ist es, ein Abrutschen der Finger während des Bearbeitungsvorgangs zu verhindern. Einerseits wird dadurch das Verletzungsrisiko verringert und andererseits wird die Gefahr einer Kontamination durch Berührung des Knochentransplantats reduziert.

Der Halteeinsatz 207 der Vorrichtung zum Präparieren eines Knochentransplantats 200 besteht aus Metall oder Kunststoff. Vorzugsweise ist der Halteeinsatz 207 aus einem Edelstahl gefertigt. Ist der Halteeinsatz 207 aus Kunststoff gefertigt, kommt vorzugsweise auch hier ein PA (Polyamid), ein PEEK (Polyetheretherketon), ein PPSU (Polyphenylensulfon) oder ein POM-C (Polyoxymethylen Copolymer) zum Einsatz. Die Materialien sind nur beispielhaft genannt und schränken die mögliche Materialauswahl nicht ein.

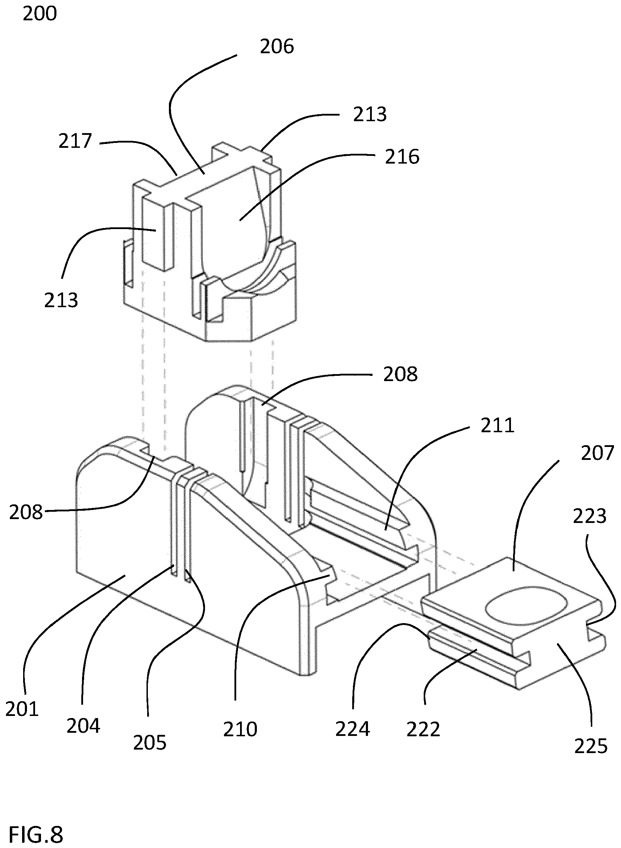

FIG. 8 zeigt das zweite Ausführungsbeispiel der erfindungsgemäßen Vorrichtung zum Präparieren eines Knochentransplantats 200. Anhand dieser Explosionsdarstellung erläutern wir im Folgenden das Verfahren zum Konfigurieren der erfindungsgemäßen Vorrichtung zum Präparieren eines Knochentransplantats 200. Vorbereitend zum Präparieren eines Knochenstücks werden die Komponenten der Vorrichtung zum Präparieren des Knochentransplantats 200 wie folgt zusammengesetzt:
In einem ersten Schritt wird der Führungseinsatz 206 in den Grundkörper 201 eingesetzt. Dies erfolgt durch Einschieben des Führungseinsatzes 206 mit seinen zwei Führungsaufnahmen 213 in die dafür vorgesehenen Ausnehmungen 208 des Grundkörpers 201. Die Anschlagsfläche 216, 217 mit dem geforderten Winkel wird dabei so ausgerichtet, dass sie den Schlitzen 204, 205 zur Führung des Schneidwerkzeugs im Grundkörper zugewandt ist.

In einem zweiten Schritt wird der Halteeinsatz 207 in den Grundkörper 201 eingesetzt. Dabei wird der Halteeinsatz 207 so positioniert, dass die stirnseitige Fläche 224, 225 mit dem geforderten Winkel in Richtung des Führungseinsatzes 206 orientiert ist. Durch die Geometrie der Führungen 222, 223 des Halteeinsatzes 207 in Verbindung mit der Geometrie der Führungsaufnahmen 210, 211 ist sichergestellt, dass der Halteeinsatz 207 nicht verkehrt herum eingesetzt werden kann. Würde ein verkehrt herum eingesetzter Halteeinsatz 207 an das zu bearbeitende Knochenstück gedrückt werden, könnte das Knochenstück durch eine ungenaue Schnittführung oder eine punktuelle Krafteinwirkung beschädigt und evtl. unbrauchbar werden.

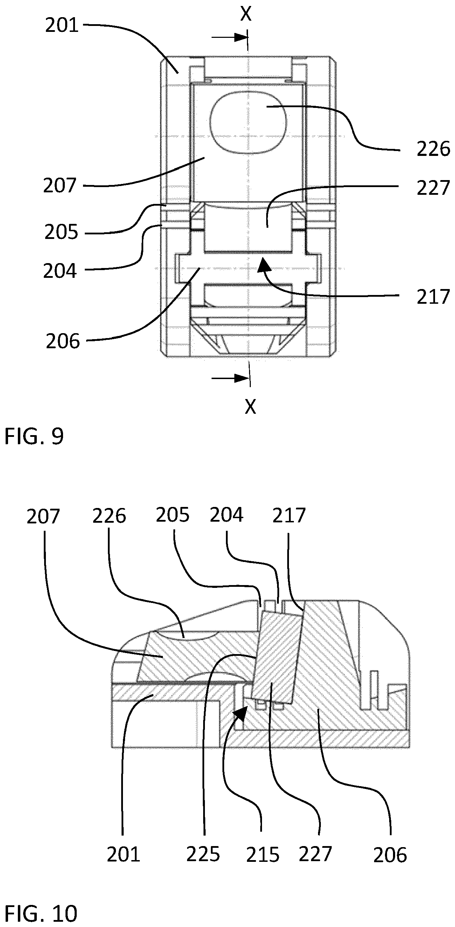

FIG. 9 zeigt das zweite Ausführungsbeispiel der erfindungsgemäßen Vorrichtung zum Präparieren eines Knochentransplantats 200 mit einem eingelegten Knochentransplantat 227. Das zu präparierende Knochentransplantat 227 liegt flächig auf der Anschlagfläche 217 des Führungseinsatzes 206 auf. Mit Hilfe des Halteeinsatzes 207 wird das Knochentransplantat 227 gegen die Anschlagsfläche 217 gedrückt. Hierzu schiebt der Anwender mit Hilfe der Fingermulde 226 den Halteeinsatz 207 in Richtung Führungseinsatz 206. Während der Bearbeitung des Knochentransplantats 227 hält der Anwender das Knochentransplantat 227 durch kontinuierliches Einleiten einer Schubkraft auf dem Halteeinsatz 207 fest. Das Bearbeiten, d. h. das Zuschneiden des Knochentransplantats 227, erfolgt über einen der Schlitze 204, 205 im Grundkörper 201. Der zu nutzende Schlitz 204, 205 wird entsprechend den Vorgaben für das Knochentransplantat 227 ausgewählt.

Zur weiteren Verdeutlichung zeigt FIG. 10 das zweite Ausführungsbeispiel der Vorrichtung zum Präparieren eines Knochentransplantats 200 mit eingelegtem Knochentransplantat 227 in Schnittdarstellung. Die Schnittebene ist in FIG. 9 mit dem Schnitt X-X markiert. Hier ist deutlich zu erkennen, wie die Auflagefläche 215 der halbkreisförmigen Ausnehmung rechtwinklig zur zugehörigen Anschlagfläche 217 ausgerichtet ist. Somit ist das Knochentransplantat 227, das vor dem Präparieren zylinderförmig ist, exakt gelagert. In diesem Beispiel ist die Vorrichtung zum Präparieren eines Knochentransplantats 200 so konfiguriert, dass der kleinere der beiden möglichen Winkel zur Bearbeitung verwendet wird. Der Führungseinsatz 206 ist so eingelegt, dass die Anschlagfläche 217 mit dem kleineren Winkel in Richtung Schlitz 204, 205 des Grundkörpers 201 weist. Dementsprechend ist der Halteeinsatz 207 so eingeschoben, dass dessen stirnseitige Fläche 225 mit dem kleineren der beiden möglichen Winkel auf dem Knochentransplantat 227 aufliegt. Mit Hilfe der Fingermulde 226 wird der Halteeinsatz 207 in Richtung Knochentransplantat 227 geschoben und dadurch festgehalten.

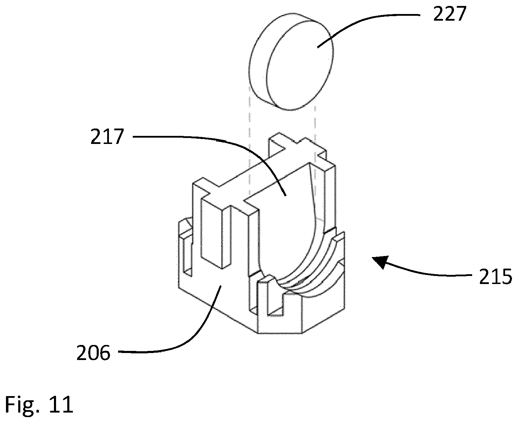

FIG. 11 veranschaulicht beispielhaft nur an einer Seite des Führungseinsatzes 206 den Zusammenhang der Formgebung des Führungseinsatzes 206 mit einem extrahierten Knochentransplantat. Die Zusammenhänge sind auf alle weiteren anwendbaren Seiten des Führungseinsatzes übertragbar. Bei dem Knochentransplantat 227 handelt es sich um eine zylinderähnliche Knochenscheibe. Der Durchmesser dieser Knochenscheibe wird bestimmt durch einen Kronenbohrer, der zum Extrahieren von Knochen, z. B. aus dem Humeruskopf, Verwendung findet und ist auf das einzusetzende Gelenksimplantat abgestimmt. Die Kreisflächen des Knochentransplantats 227 sind durch das Extraktionsverfahren senkrecht zu dessen Zylinderwand ausgerichtet. Die halbkreisförmige Ausnehmung 215 des Führungseinsatzes 206 hat einen dem Knochentransplantat 227 entsprechenden Durchmesser. Die Wandung der halbkreisförmigen Ausnehmung 215 und die zugehörige Anschlagfläche 217 stehen, entsprechend der Zylinderform des Knochentransplantats, senkrecht zueinander.

Durch die halbkreisförmige Gestaltung der Ausnehmung 215 ist es möglich, das Knochentransplantat 227, wie in FIG. 11 gezeigt, von oben in den Führungseinsatz 206 einzuschieben. Dies kann auch bei einer zusammengesetzten Vorrichtung 200 zum Präparieren eines Knochentransplantats 227 erfolgen.

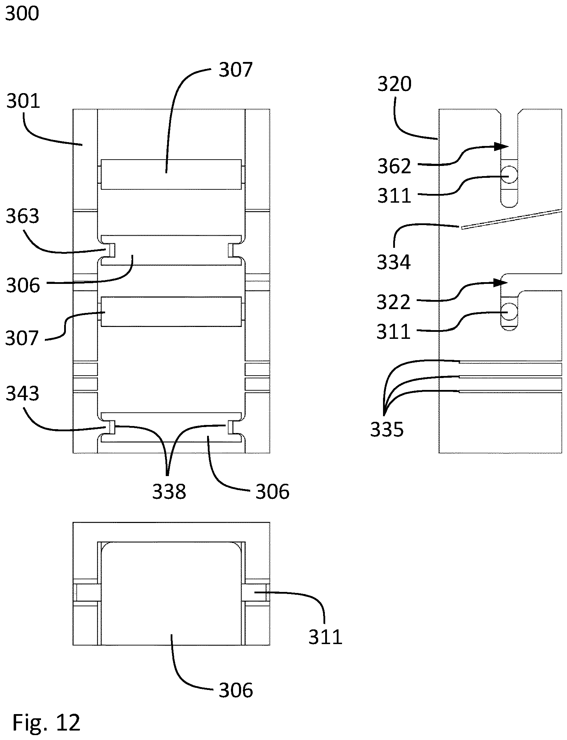

FIG. 12 zeigt ein drittes Ausführungsbeispiel der erfindungsgemäßen Vorrichtung zum Präparieren eines Knochentransplantats 300 mit einem Grundkörper, einem Führungseinsatz 306 und einem Halteeinsatz 307 im Dreitafelbild. Die abgebildete Vorderansicht zeigt die Vorrichtung zum Präparieren eines Knochentransplantats 300 von oben.

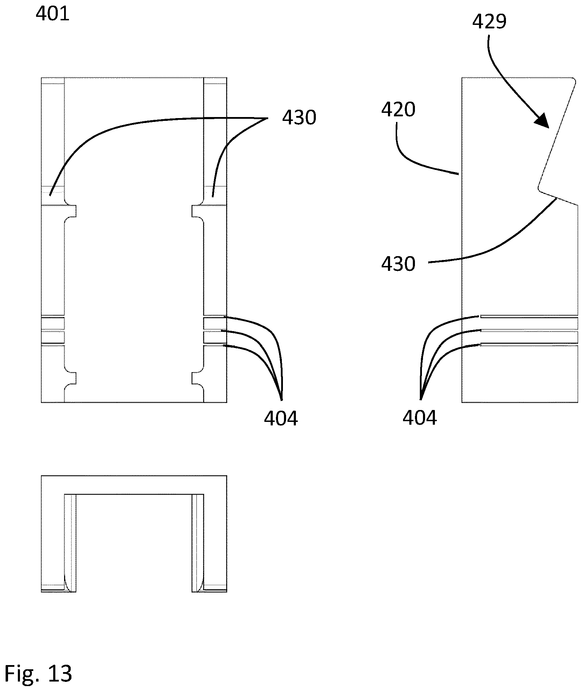

Der Grundkörper 301 enthält zum einen drei senkrecht zur Auflagefläche 320 des Grundkörpers 301 verlaufende Schlitze 335. Zum anderen enthält der Grundkörper 301 einen schräg verlaufenden Schlitz 334. Dabei ist der Winkel auf den in den Grundkörpers 301 eingelegten Führungseinsatz 306 bezogen. Die Schlitze 334, 335 sind so ausgelegt, dass darin ein spanabhebendes Werkzeug präzise geführt wird. Die Anzahl der beispielhaft in FIG. 13 dargestellten Schlitze 334, 335 können je nach Anforderung variieren. In diesem dritten Ausführungsbeispiel lassen sich somit Knochenstücke mit drei unterschiedlichen Längen und einem durch den schräg verlaufenden Schlitz 334 definierten Winkel präparieren. Der Winkel des schräg verlaufenden Schlitzes 334 kann, abhängig von der Anforderung an das Knochentransplantat, einen Winkel zwischen 0° und 45° aufweisen. In diesem Ausführungsbeispiel beträgt der Winkel des schräg verlaufenden Schlitzes 334 10°.

Der Grundkörper 301 enthält zwei Führungsaufnahmen 343, 363. Die Führungsaufnahmen 343, 363 dienen zur Führung des Führungseinsatzes 306. Um eine schiebbare Führung des Führungseinsatzes 306 in einer der Führungsaufnahmen 343, 363 sicherzustellen, enthält der Führungseinsatz 306 Ausnehmungen 338, die mit den Führungsaufnahmen 343, 363 korrespondierenden. Die erste Führungsaufnahme 343 wird verwendet, wenn das zu präparierenden Knochenstück auf die für das Knochentransplantat geforderte Länge gekürzt wird.
Wenn eine Fläche des zu präparierenden Knochenstück mit einem Winkel präpariert werden muss, wird der Führungseinsatz 306 in die zweite Führungsaufnahme 363 eingeschoben.

Des Weiteren zeigt FIG. 12 schlitzförmige Ausnehmungen 322, 362 im Grundkörper 301. Diese schlitzförmigen Ausnehmungen 322, 362 sind zur Führung des Halteeinsatzes 307 ausgelegt. Der Halteeinsatz 307 enthält zylinderförmige Zapfen 311. Die zylinderförmigen Zapfen 311 sind im Durchmesser so ausgelegt, dass sie in Verbindung mit den schlitzförmigen Ausnehmungen 322, 362 eine schiebbare Führung ergeben. Dabei ist die schiebbare Führung so ausgelegt, dass ein notwendiges Spiel die Präzision der Vorrichtung zum Präparieren eines Knochentransplantats 300 nicht beeinträchtigt.

Eine erste schlitzförmige Ausnehmung 322 ist in L-Form ausgeführt. Die L-Form macht es möglich, den Halteeinsatz 307 von oben in den Grundkörper 301 einzuführen. Mit Hilfe des Halteeinsatzes 307 wird das zu präparierende Knochenstück an den Führungseinsatz 306 gedrückt. Dabei ist der Führungseinsatz 306 in der Position zum Kürzen des Knochenstücks in die Führungsaufnahme 343 eingeschoben. Eine zweite schlitzförmige Ausnehmung 362 ist geradlinig von einem Ende des Grundkörpers 301 ausgeführt. Hier wird der Halteeinsatz 307 von dem entsprechenden Ende in den Grundkörper 301 eingeführt. Der Führungseinsatz 306 ist in diesem Fall in die Position zum Präparieren eines Winkels am Knochenstück in die Führungsaufnahme 363 eingeschoben. Mit Hilfe des Halteeinsatzes 307 wird das zu präparierende Knochenstück an den in die Führungsaufnahme 363 eingesetzten Führungseinsatz 306 gedrückt.

Der Grundkörper 301, der Halteeinsatz 306 und/oder der Führungseinsatz 307 der Vorrichtung zum Präparieren eines Knochentransplantats 300 bestehen aus Metall oder Kunststoff. Vorzugsweise ist der Grundkörper 301, der Halteeinsatz 306 und/oder der Führungseinsatz 307 aus einem Edelstahl gefertigt. Ist der Grundkörper 301, der Halteeinsatz 306 und/oder der Führungseinsatz 307 aus Kunststoff gefertigt, kommt vorzugsweise ein PA (Polyamid), ein PEEK (Polyetheretherketon), ein PPSU (Polyphenylensulfon) oder ein POM-C (Polyoxymethylen Copolymer) zum Einsatz. Die Materialien sind nur beispielhaft genannt und schränken die mögliche Materialauswahl nicht ein.

FIG. 13 zeigt einen Grundkörper 401 eines vierten Ausführungsbeispiels der Vorrichtung zum Präparieren eines Knochentransplantats im Dreitafelbild. In der Seitenansicht ist der Grundkörper 401 mit drei Schlitzen 404 gezeigt, wobei die Anzahl der Schlitze 404 nicht auf drei beschränkt ist. Diese Schlitze 404 verlaufen senkrecht zur Auflagefläche 420 des Grundkörpers 401. Mit Hilfe der Schlitz 404 lässt sich ein Knochentransplantat in der geforderten Dicke erstellen. Die Dicke des Knochentransplantats wird vom Präparator durch Auswahl einer der Schlitze 404 mit einem geeigneten Abstand zum Führungseinsatz (hier nicht dargestellt) festgelegt.

Des Weiteren enthält der Grundkörper 401 einen Bereich zum Präparieren einer abgeschrägten Fläche 429 am Knochentransplantat. Zum Präparieren einer abgeschrägten Fläche 429 ist eine Planfläche 430 enthalten. Diese Planfläche 430 dient zum Führen eines spanabhebenden Werkzeugs und ist Lotrecht zur Auflagefläche 420 des Grundkörpers 401 mit einem Winkel versehen. Der Winkel der Planfläche 430 kann, abhängig von der Anforderung an das Knochentransplantat, einen Winkel zwischen 0° und 45° aufweisen. Bei dem Grundkörper 201 in diesem Ausführungsbeispiel hat die Planfläche 430 vorzugsweise einen Winkel von 20°.

Der Grundkörper 401 der Vorrichtung zum Präparieren eines Knochentransplantats des vierten Ausführungsbeispiels besteht aus Metall oder Kunststoff. Vorzugsweise ist der Grundkörper 401 aus einem Edelstahl gefertigt. Ist der Grundkörper 401 aus Kunststoff gefertigt, kommt vorzugsweise ein PA (Polyamid), ein PEEK (Polyetheretherketon), ein PPSU (Polyphenylensulfon) oder ein POM-C (Polyoxymethylen Copolymer) zum Einsatz. Die Materialien sind nur beispielhaft genannt und schränken die mögliche Materialauswahl nicht ein.

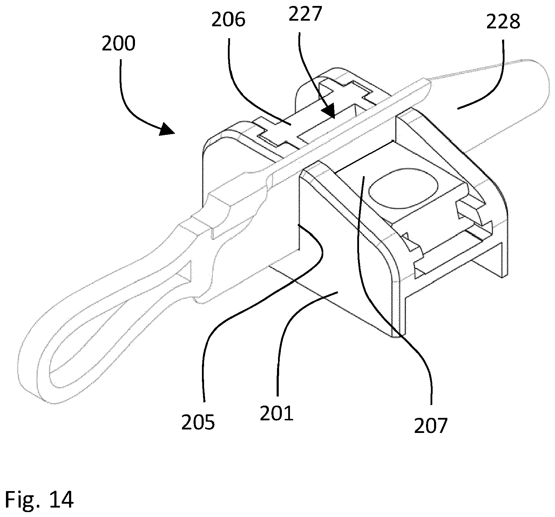

FIG. 14 zeigt die Anwendung der erfindungsgemäßen Vorrichtung zum Präparieren eines Knochentransplantats 100, 200 beispielhaft. Ein Knochentransplantat 227 wird bei der erfindungsgemäßen Vorrichtung und den zugehörigen Verfahren außerhalb des menschlichen oder tierischen Körpers eingesetzt. Hier kommt die sogenannte "Backtable Preparation" mit ihren spezifischen Vorteilen zum Einsatz. Das Präparieren außerhalb des Körpers des Patienten auf dem "Backtable" führt zu einer erheblichen Steigerung der Präzision des präparierten Knochentransplantats 227. Außerdem ist die Belastung für den Patienten wesentlich geringer, da die Region, aus der das Knochentransplantat 227 entnommen wird, nicht so ausgerichtet werden muss, dass eine exakte Schnittführung möglich ist und somit eine weitere Belastung des Muskel- und Sehnenapparats vermieden wird.

Wie in FIG. 14 zu erkennen ist, wird das Knochentransplantat 227 in der Vorrichtung zum Präparieren eines Knochentransplantats 200 mit Hilfe des in den Grundkörper 201 eingesetzten Führungseinsatzes 206 und des in den Grundkörper 201 eingesetzten Halteeinsatzes 207 fixiert. Mit einem spanabhebenden Werkzeug 228, hier einer Handsäge, wird das zu präparierende Knochentransplantat 227 zugeschnitten. Hierzu wird das spanabhebende Werkzeug 228 in den Schlitz 205 des Grundkörpers 201, der die für das Knochentransplantat 227 vorgesehene Länge vorgibt, eingeführt. Nun wird das Knochentransplantat 227 abgelängt. Ist der Präparationsvorgang abgeschlossen, wird das Knochentransplantat 227 aus der Vorrichtung zum Präparieren eines Knochentransplantats 200 entnommen. Nun steht das Knochentransplantat 227 in der geforderten Form zur weiteren Verwendung zur Verfügung.

Die Vorrichtung zum Präparieren eines Knochentransplantats 200 wird anschließend zerlegt, gereinigt und desinfiziert. Durch die einzigartige Konstruktion der Vorrichtung zum Präparieren eines Knochentransplantats 200 ist der Reinigungs- und Desinfektionsprozess mit einer außergewöhnlich hohen Prozesssicherheit durchführbar.

Alle beschriebenen und/oder gezeichneten Merkmale können im Rahmen der Erfindung vorteilhaft miteinander kombiniert werden. Die Erfindung ist nicht auf die beschriebenen Ausführungsbeispiele beschränkt.

## Patentansprüche

1. Vorrichtung zum Präparieren eines Knochentransplantats (100, 200) außerhalb des menschlichen oder tierischen Körpers mit:
einem Grundkörper (101, 201) und zumindest einem Halteelement (102, 202), wobei das zumindest eine Halteelement (102, 202) in den Grundkörper (101, 201) eingesetzt ist,
wobei das erste Halteelement (102) einen Führungseinsatz (106) formt;
wobei der Grundkörper (101, 201) eine Anschlagsfläche (103) umfasst;
wobei der Grundkörper (101, 201) mindestens einen Schlitz (104, 105, 204, 205) zur Führung eines spanabhebenden Werkzeugs (228) enthält;
wobei die Anschlagsfläche (103) einen Winkel relativ zu dem mindestens einen Schlitz (104, 105, 204, 205) zur Führung eines spanabhebenden Werkzeugs (228) aufweist, und
wobei ein Halteeinsatz (207) einen Winkel relativ zu dem mindestens einen Schlitz (104, 105, 204, 205) zur Führung eines spanabhebenden Werkzeugs (228) aufweist, der dem Winkel der Anschlagsfläche entspricht.

2. Die Vorrichtung nach Anspruch 1, wobei der Grundkörper (101, 201) mindestens eine Planfläche (430) zur Führung eines spanabhebenden Werkzeugs (228) enthält.

3. Die Vorrichtung nach Anspruch 1 oder 2,
wobei der Halteeinsatz (207) eine erste Seite (224) und eine zweite Seite (225) aufweist,
wobei die erste Seite (224) einen Winkel relativ zu dem mindestens einen Schlitz (104, 105, 204, 205) aufweist und
wobei die zweite Seite (225) einen zweiten Winkel relativ zu dem mindestens einen Schlitz (104, 105, 204, 205) aufweist, der sich von dem Winkel relativ zu dem mindestens einen Schlitz (104, 105, 204, 205) unterscheidet.

4. Die Vorrichtung nach Anspruch 3,
wobei die erste Seite (224) der zweiten Seite (225) gegenüberliegt.

5. Die Vorrichtung nach einem der Ansprüche 1 bis 4,
wobei der Halteeinsatz (207) mindestens zwei Führungen (222, 223) aufweist, wobei die mindestens zwei Führungen (222, 223) geometrisch unterschiedlich ausgeführt sind und
wobei der Grundkörper (101, 201) mindestens zwei Führungsaufnahmen (210, 211) zur Aufnahme der mindestens zwei Führungen (222, 223) enthält.

6. Die Vorrichtung nach Anspruch 5,
wobei die mindestens zwei Führungen (222, 223) auf gegenüberliegenden Seiten des Halteeinsatzes (207) angeordnet sind.

7. Die Vorrichtung nach Anspruch 5 oder Anspruch 6,
wobei die mindestens zwei Führungen (222, 223) geometrisch unterschiedlich sind, indem eine erste Führung (222) der mindestens zwei Führungen (222, 223) weiter von einem Boden des Halteeinsatzes entfernt angeordnet ist als eine zweite Führung (223) der mindestens zwei Führungen (222, 223).

8. Die Vorrichtung nach einem der Ansprüche 5 bis 7,
wobei der Grundkörper (101, 201) mindestens zwei Führungsaufnahmen (210, 211) enthält und
wobei die mindestens zwei Führungsaufnahmen (210, 211) so ausgestaltet sind, dass sie die mindestens zwei Führungen (222, 223) aufnehmen.

9. Die Vorrichtung nach einem der Ansprüche 1 bis 8,
wobei die Anschlagsfläche (103) eine halbkreisförmige Ausnehmung (214) enthält.

10. Die Vorrichtung nach Anspruch 9,
wobei eine Achse der halbkreisförmigen Ausnehmung (214) so abgewinkelt ist, dass sie dem Winkel der Anschlagsfläche (103) entspricht.

11. Die Vorrichtung nach einem der Ansprüche 1 bis 10,
wobei der Halteeinsatz (207) mindestens eine Fingermulde (226) enthält.

12. Ein Verfahren zum Konfigurieren einer Vorrichtung zum Präparieren eines Knochentransplantats außerhalb des menschlichen oder tierischen Körpers nach einem der Ansprüche 1 bis 11, mit dem Verfahrensschritt:
Einsetzen des zumindest einen Halteelements (102, 202) in den Grundkörper (101, 201).

13. Das Verfahren nach Anspruch 12,
wobei der Halteeinsatz (207) in den Grundkörper (101, 201) eingesetzt wird.
